Europäisches Patentamt

European Patent Office

Office européen des brevets

(11) Publication number: **0 419 393 A1**

# EUROPEAN PATENT APPLICATION

(21) Application number: **90500027.9**

(22) Date of filing: **22.03.90**

(51) Int. Cl.⁵: **A61B 3/06**

(30) Priority: **19.09.89 ES 8903165**

(43) Date of publication of application:
**27.03.91 Bulletin 91/13**

(84) Designated Contracting States:
**AT BE CH DE DK FR GB GR IT LI LU NL SE**

(71) Applicant: **ORGANIZACION NACIONAL DE CIEGOS ESPANOLES - ONCE -**
**c./ Prado, 24**
**E-28014 Madrid(ES)**

(72) Inventor: **Navarro Belsue,Rafael, Instituto de Optica**
**Daza de Valdés, c/o Serrano, 121**
**E-28006 Madrid(ES)**
Inventor: **Garcia, Perez Manuel, Instituto de Optica**
**Daza de Valdés, c/o Serrano, 121**
**E-28006 Madrid(ES)**
Inventor: **Losada Binue, Angeles, Instituto de Optica**
**Daza de Valdés, c/o Serrano, 121**
**E-28006 Madrid(ES)**

(74) Representative: **Carpintero Lopez, Francisco HERRERO & ASOCIADOS, S.L. Alcalá, 21**
**E-28014 Madrid(ES)**

(54) **Computerized system for measuring sensitiveness function to contrast specification.**

(57) The system allows the threshold contrast of sinusoidal luminance distributions of different frequencies to be determined, thereby allowing the sensitiveness function to contrast of the visual system to be obtained. To generate the sights a PC computer (1) and a digital video control (2) are used, the latter allowing presentation of the sights in real time, on a TV monitor (3). Contrast variation and processing and obtention of results is effected through the suitable programmes where the patient's answers are introduced through a mouse (4).

# COMPUTERIZED SYSTEM FOR MEASURING SENSITIVENESS FUNCTION TO CONTRAST

## OBJECT OF THE INVENTION

This invention relates, as stated in the title hereof, to a computerized system for measuring the sensitiveness function to contrast by determining the threshold of sinusoidal luminance distributions of different frequencies.

## BACKGROUND OF THE INVENTION

The sensitiveness function to contrast was first defined and measured by Campbell and Green, as a psychophysical equivalent of an optical system transfer function. In other words, the sensitiveness function to contrast characterizes the quality level, as to transmission of information, of the visual system, for given adaptation, accommodation, and other conditions. Such function is usually obtained by providing sinusoidal sights of different spatial frequency, varying their contrast until the threshold value for which they cease to be visible is obtained. The threshold value is obtained through the subjective answers provided by the patient.

To date, several systems have been developed, some of them commercial, essentially based on two different principles. In some cases sinusoidal sights are obtained using electronic frequency generators, that modulate the inlet signal of a video monitor. In other cases, when a quick and scarcely accurate estimate is required, sinusoidal sights printed on paper or posters are used.

## DESCRIPTION OF THE INVENTION

The foregoing systems have some advantages that can be joined together using a wholly digital system such as that set forth herein.

Specifically, the computerized system for measuring the sensitiveness function to contrast comprises a PC compatible computer, a digital video control connected to the computer, a high resolution and multisynchronous television monitor and a mouse with at least two buttons, and that can also have an arithmetical co-processor to work in real time and a mirror, to be placed at 45°, with an elliptical hole to observe a circular area in the monitor centre. This system must be duly programmed both to generate and present the stimuli and to implement the adjustment methods, forced election, and so on.

Given the present great upsurge and low-cost of IBM, PC and compatible personal computers, the system proposed, developed on a computer of this type, may hugely facilitate dissemination among specialists, who normally use a computer for other matters.

## DESCRIPTION OF THE DRAWINGS

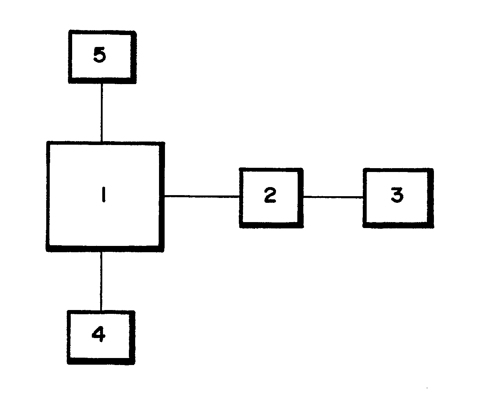

In order to complement the description being made and to assist a better understanding of the characteristics of the invention, a sheet of drawings is attached to the present specification as an integral part thereof its only figure showing, in an illustrative and non-limiting manner, a block diagram of the computerized system for measuring sensitiveness function to contrast set forth by the invention.

## PREFERRED EMBODIMENT OF THE INVENTION

In the light of the figure it may be observed that the computerized system for measuring the sensitiveness function to contrast set forth comprises a personal computer (1), a digital video control (2), connected to the computer, a TV monitor (3), a mouse (4) with at least two buttons and an arithmetic co-processor (5) to work in real time.

In order to measure the sensitiveness function to contrast, it is necessary to present a visual test comprising sinusoidal sights of different spatial frequency, orientation and contrast. The arithmetic expression for sight intensity is as follows:

$I(X,Y) = I_0\, 1 + C \cos 2 f (x \cos \theta + y \sin \theta)$

where x,y are spatial coordinates, $I_0$ is constant, C is the contrast, f is the frequency and $\theta$ is the orientation. To generate this type of test, the use of a digital video control (2) (digital-analogical video converter) is proposed connected to a personal computer (1), using a fast algorithm and an arithmetic co-processor (5) (to work in real time) and the use of a monitor (3), such multisynchronism allowing the stimulus to be presented with non-interlocked sweeping, thereby avoiding blinking problems etc.

It is moreover necessary to collect and analyze the patient's reply. There are several methods, mainly two:

a). The actual patient adjusts or chooses the test contrast, that he or she deems threshold (from viewing to not viewing the bands, and vice versa). This method depends largely on the patient's opinion and shall generally be called adjustment.

b). Forced election between two alternatives. Two stimuli are presented, one with sights and another one with uniform beach, in random order, and the patient must try to guess in which of the two presentations the test (sights) was. The threshold contrast is found varying the test contrast, depending on the patient's success or failure: two correct answers in a row result in lowering the contrast in the following presentation, whereas an error makes the contrast to be increased. This method is called ladder, converging (contrast increases or decreases must gradually diminish) and leads to a threshold contrast value for which the number of correct answers is 75%.

The system proposed may optionally use both methods, specifically the use of a mouse (4) with two buttons, connected to the computer (1). The actual patient shall press on one button, either to increase the test contrast or to inform the computer that the stimulus appeared in the first place; the other button will do the opposite. Given the high versatility of the digital systems, two or more operation systems are moreover proposed. Swiftly, with few presentations, when a less accurate estimate is required and a more accurate method, slower for stricter tests. The computer also facilitates all subsequent treatment, as well as data presentation. The system thus described may be easily adapted for obtaining threshold contrasts of any other type of stimulus, optotypes, etc.

Since a high resolution digital image comprises at least 512 x 512 pixels, calculation of all these points, and more so in the case of sinusoidal functions (important) is long. In order to be able to work in real time, and that there are no continual and rather long waiting intervals between presentations, a fast algorithm has been designed allowing generation and presentation of the variable sinusoidal sights for frequency, orientation and contrast (see formula), in real time. The method consists in generating a single row, through a fast algorithm, comprising calculating only the first value of the sine and the cosine, the rest being obtained through recursive trigonometric expressions (double angle). Once the first row has been obtained, the others are obtained by replicate. The different orientations (specifically $0°$, $\pm 26.56°$, $\pm 45°$, $\pm 63°$ and $90°$), are attained transferring each row or, $\pm 1/2$, $\pm 1$ or $\pm 2$ pixels ($90°$ involves replacing rows by columns) in respect of the previous one (other angles may be achieved with more complex fractionary translations, $3/4$, $1/3$, etc. but are unnecessary, since the eight orientations mentioned serve to perfectly characterize the sensitiveness function to contrast). Thus, it is only necessary to calculate a single row. This calculation should be made with a fast algorithm.

Finally, the adjustment methods, forced election between two alternatives, etc. are fitted in a single apparatus. Specifically, within the ladder method, use of two variants to control accuracy or time measurement is proposed. Thus, a modality would be to predetermine the number of presentations to complete a ladder (for fast estimates that do not require high accuracy). The other modality comprises presetting the accuracy or the tolerable error interval (that can be programmed as an option, as appropriate), the number of presentations required to reach such level then being made.

It is not considered necessary to extend the present description any further for an expert in the art to understand the scope of the invention and the advantages derived therefrom.

The materials, shape, size and arrangement of the elements may vary, provided this does not imply a modification in the essence of the characteristics of the invention.

The terms used to describe the present specification should be understood to have a wide and non-limiting meaning.

## Claims

1.- Computerized system for measuring the sensitiveness function to contrast, aiming to measure the sensitiveness function to contrast by determining the threshold contrast of distributions of sinusoidal luminance of different frequencies, essentially characterized because it comprises the functional association of a PC compatible computer (1), a digital video control (2), a high resolution TV monitor (3), a mouse (4) with at least two buttons and an arithmetic co-processor (5).

European
Patent Office

**EUROPEAN SEARCH
REPORT**

Application Number

**EP 90 50 0027**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 096 325 (INTERZEAG AG) <br> * figure 6 * <br> – – – | 1 | A 61 B 3/06 |
| A | EP-A-0 228 936 (ETAT-FRANCAIS) <br> * page 2, lines 7-10,23-29; abstract; figure 1 * <br> – – – | 1 | |
| A | US-A-4 511 228 (H.E. VON GIERKE) <br> * abstract; figure 1 * <br> – – – – – | 1 | |

| | TECHNICAL FIELDS SEARCHED (Int. Cl.5) |
|---|---|
| | A 61 B 3/00 |

The present search report has been drawn up for all claims

| Place of search | Date of completion of search | Examiner |
|---|---|---|
| Berlin | 17 December 90 | WEIHS J.A. |